# EUROPEAN PATENT SPECIFICATION

(11) **EP 3 948 406 B1**
(45) Date of publication and mention of the grant of the patent: **02.07.2025**
(21) Application number: 20719720.3
(22) Date of filing: 24.03.2020
(51) Int. Cl.: G16H 20/30, G16H 20/70, G16H 40/63, G16H 50/20, G02C 7/14, A61B 5/00, A61B 5/16, G02B 26/08, G02C 7/08, G02C 7/10, G02B 27/01, A61H 5/00, A61M 21/00

(54) **A SYSTEM AND A METHOD FOR ENHANCING OR REHABILITATING THE COGNITIVE SKILLS OF A SUBJECT**
SYSTEM UND VERFAHREN ZUR FÖRDERUNG ODER REHABILITIERUNG DER KOGNITIVEN FÄHIGKEITEN EINES SUBJEKTS
SYSTÈME ET PROCÉDÉ POUR AMÉLIORER OU RESTAURER LES COMPÉTENCES COGNITIVES D'UN SUJET

(30) Priority: 25.03.2019 IT 201900004269
(43) Date of publication of application: 09.02.2022
(73) Proprietor: Restorative Neurotechnologies S.r.l., 90143 Palermo (IT)
(72) Inventor: OLIVERI, Massimiliano, 90144 Palermo (IT)
(74) Representative: Savoca, Agatino
(86) International application number: PCT/IB2020/052737
(87) International publication number: WO 2020/194180

(56) References cited:
- US-A1- 2015 320 350
- US-A1- 2016 349 533
- US-A1- 2018 143 456
- ROSSETTI YVES ET AL: "Prism adaptation: From rehabilitation to neural bases", CORTEX, vol. 111, 28 January 2019 (2019-01-28), AMSTERDAM, NL, pages A1 - A6, XP055779597, ISSN: 0010-9452, Retrieved from the Internet <URL:http://dx.doi.org/10.1016/j.cortex.2019.01.002> DOI: 10.1016/j.cortex.2019.01.002
- PIEMONTESE ANTONIO: "Da Harvard a Palermo, storia degli occhiali che aiutano chi soffre di ictus e dislessia", 10 February 2019 (2019-02-10), XP055812820, Retrieved from the Internet <URL:https://startupitalia.eu/69372-20190210-occhiali-riabilitazione-neurologica> [retrieved on 20210610]
- ANONYMOUS: "Ophthalmic Prism Adapter Set | Fresnel Prism Lens", 7 January 2016 (2016-01-07), XP055646743, Retrieved from the Internet <URL:https://www.fresnel-prism.com/product/professional-products/professional-examination-tools/professional-ophthalmic-prism-adapter/ophthalmic-prism-adapter-set/> [retrieved on 20191126]
- ISABEL TISSIERES ET AL: "For Better or Worse: The Effect of Prismatic Adaptation on Auditory Neglect", NEURAL PLASTICITY, vol. 2017, 12 September 2017 (2017-09-12), pages 1 - 11, XP055647643, ISSN: 2090-5904, DOI: 10.1155/2017/8721240
- RESTORATIVE NEUROTECHNOLOGIES SRL: "Mindlenses professional | La riabilitazione cognitiva", 6 December 2019 (2019-12-06), pages 1, XP054980570, Retrieved from the Internet <URL:https://www.youtube.com/watch?v=xN0jIZmKcUU> [retrieved on 20200615]
- GABRIELE CHIARAMONTE: "LA SOLUZIONE mindlenses professional", 13 November 2019 (2019-11-13), XP055704916, Retrieved from the Internet <URL:https://www.restorativeneurotechnologies.com/documents/Presentazione%20mindlensrs_professional.pdf> [retrieved on 20200615]

## Description

### Technical field

The present invention generally relates to the field of medical rehabilitation equipment; in particular, the invention relates to a system for enhancing cognitive functions and/or modulating the immune responses of a patient.

### Prior art

The incidence and extent of disabilities related to neurological problems are known. Only in Europe it is estimated that, to date, 38% of the population suffers from neurological disorders such as epilepsy, Parkinson's disease, Alzheimer's disease, Multiple Sclerosis, stroke and headaches. These are diseases with a significant impact not only on the lives of people who are affected, but also on that of their family members, and which have considerable economic and social-welfare consequences.

These diseases are extremely important. Suffice it to say that in Europe the burden of neurological diseases is equal to about a third of general health expenditure.

For these reasons, and for the spread and impact they have on the world population, neurological diseases represent real social diseases. Numerous international epidemiological studies predict a dramatic increase in the number of cases of people with dementia over the next two decades, for the vast majority concentrated in developing countries.

On the other hand, the scientific literature suggests an association between the activity of specific parts of the brain, and associated cognitive processes, and the immune response.

It is necessary to implement rehabilitation practices that help the patient to maintain or regain his/her skills.

There are wearable devices for monitoring physiological functions, capable of measuring signals such as heart rate, respiration, movement, electrodermal activity, but not of directly modulating cognitive functions in a non-invasive manner.

On the other hand, the procedures used for the rehabilitation/enhancement of these functions are based on the repetition of exercises, also computerized, which exert an indirect effect on the plasticity of the brain and therefore require very intensive applications and for long periods of time.

Procedures are known based on the application of prisms for the correction of strabismus, diplopia, ocular convergence insufficiency, visual field disturbances (hemianopsia) and spatial attention disturbances following unilateral brain injuries, more frequently at the expense of the right cerebral hemisphere.

Notoriously, an optical prism is a transparent means delimited by two flat, but not parallel, surfaces that intersect to form a refractive angle. The thinner edge of the prism, where these refractive surfaces intersect, is called the apex, while the thicker edge is the base. A beam of light rays passing through the prism undergoes a deviation towards its base, generating an apparent displacement phenomenon of an object towards the apex of the prism.

An example of a solution that exploits the use of prismatic lenses to evaluate the degree of deviation between a visual target stimulus and an aiming movement by a user is known from document US 2015/320350 A1. Another system is presented in "LA SOLUZIONE mindlenses professional" (Gabriele Chiaramonte, 2019-11-13).

However, this solution is designed only to detect dysfunctional movements of the user due to brain disorders, and to distinguish their origin, but it does not lend itself to any rehabilitative intent. In fact, a system according to the aforementioned prior art document is limited to recording the deviation between the aiming movements of the user and the visual target stimulus, without inducing any adaptation in the user.

Although therefore procedures that employ prisms for the treatment of a plurality of disorders are known, it does not appear that any exist for the rehabilitation and/or enhancement of important cognitive functions, such as short and long term memory and language. In fact, there are no systems and procedures that respond to these needs, nor systems or procedures that exploit cognitive enhancement to modulate the immune response.

### Summary of the invention

An object of the present invention is to overcome the aforementioned problems.

To obtain this result, the present invention proposes a system according to claim 1, for non-invasive modulation of a wide range of cognitive functions, such as attention, language, motor skills, short and long-term memory, through the induction of a "perturbation" of a physiological signal (vision), induced by a wearable optical device which rotatably supports at least one prismatic lens.

This spatial perturbation pushes the nervous circuits to recalibrate to compensate for the effects thereof. This recalibration triggers brain plasticity processes, the specificity of which depends on the procedure/task that the subject performs. The cognitive task performed can be administered by a software application operating in association with the system.

It is thus possible to induce an adaptation step in the user (during the induction of spatial distortion by means of the wearable optical device) and a post-adaptation phase (spatial post-distortion), at the end of which a beneficial conditioning of the subject's abilities will have been obtained.

A possible variant of the device provides for the integration of the prism in a virtual reality viewer, in order to allow a more immersive experience in spatial distortion.

A further variant of the device contemplates the use of colored prismatic lenses (for example, orange), in order to filter the light of a certain wavelength, and optimize the enhancement of specific cognitive functions (for example, those associated with the wavelength between 625 and 585 nm).

Below is an example of a rehabilitation procedure wherein a system according to the invention can be used, to illustrate the technical effects and achievable advantages thereof. In fact, the subject may be asked to make aiming movements (with a finger, a mouse, etc.) towards stimuli of different types, displayed for example on a screen (which can be a computer monitor, a portable device such as tablet or smartphone, etc.), conveniently in a central position or lateralized to the right or left by a variable angle (suitably up to 21°). The subject will be invited to overcome the spatial deviation induced by the prism and hit the target as precisely as possible. The aiming movements can be conveniently carried out from a starting position in the central space (0°), approximately corresponding to the median sagittal plane of the trunk.

The system is configured in such a way that the subject is led to make aiming movements towards central stimuli, in the right space or in the left space. Depending on the device used, aiming movements may be performed using a mouse, or a finger of the hand in the case of using devices with a touch screen. The amount of deviations, measured in degrees of visual angle, may for example be recorded by the same application that displays visual stimuli.

The stimuli to be reached may be of different types, depending on the cognitive function to be enhanced/rehabilitated: circular shaped stimuli, alphanumeric characters, numbers, words, etc.

Conveniently, the presentation of each stimulus in the various positions of the space is timed, with the disappearance of the stimulus when the subject reaches it with the movement made or after a variable time interval and the appearance of the next stimulus after a variable time interval.

Conveniently, a system according to the present invention may also be used in a post-adaptation step, wherein the subject can perform aiming movements in a condition in which spatial distortion is no longer present, for example movements towards stimuli located at the center of the display screen (0°), towards stimuli located on the right (suitably, up to 21° lateralization), towards stimuli located on the left (suitably, up to 21° lateralization). As a result of the previous adaptation to the movement of the image in the visual field, there will be a phenomenon of shifting attention towards the space opposite to that of the initial deviation of the visual field. For example, in the case of using prismatic lenses with apex positioned to the right and apparent movement of an image to the right, in the post-adaptation step it will be possible to register a deviation of the subject's attention, and consequently of his/her aiming movements towards visual target stimuli, to the left. Opposite deviation (i.e. towards the right space) will be noticed in the case of initial adaptation with prismatic lenses with apex positioned to the left and apparent movement of an image to the left.

In addition, the system may be associated with software that presents stimuli in different devices such as computers, tablets, smartphones, etc. The software may select the type of stimuli to be presented according to the cognitive function to be enhanced, while the position of the base and apex of the prismatic lenses may be set directly by the user.

The system object of the present invention, allowing the induction of a spatial distortion according to the modes that will be better specified hereinafter, allows enhancing a wide range of impaired cognitive abilities following brain pathologies, such as head injury, stroke, developmental disorders such as dyslexia and dyscalculia, acting more directly on the plasticity of the brain than existing rehabilitation procedures, increasing the effectiveness and reducing the time of cognitive rehabilitation.

The above and other objects and advantages are achieved, according to an aspect of the invention, by a system for cognitive enhancement or rehabilitation having the features defined in claim 1. Preferred embodiments of the invention are defined in the dependent claims.

### Brief description of the drawings

The functional and structural features of some preferred embodiments of a system for cognitive enhancement or rehabilitation according to the invention will now be described. Reference will be made to the accompanying drawings, wherein:
- figure 1 is a schematic perspective view of a wearable optical instrument forming part of the system according to an embodiment of the present invention;

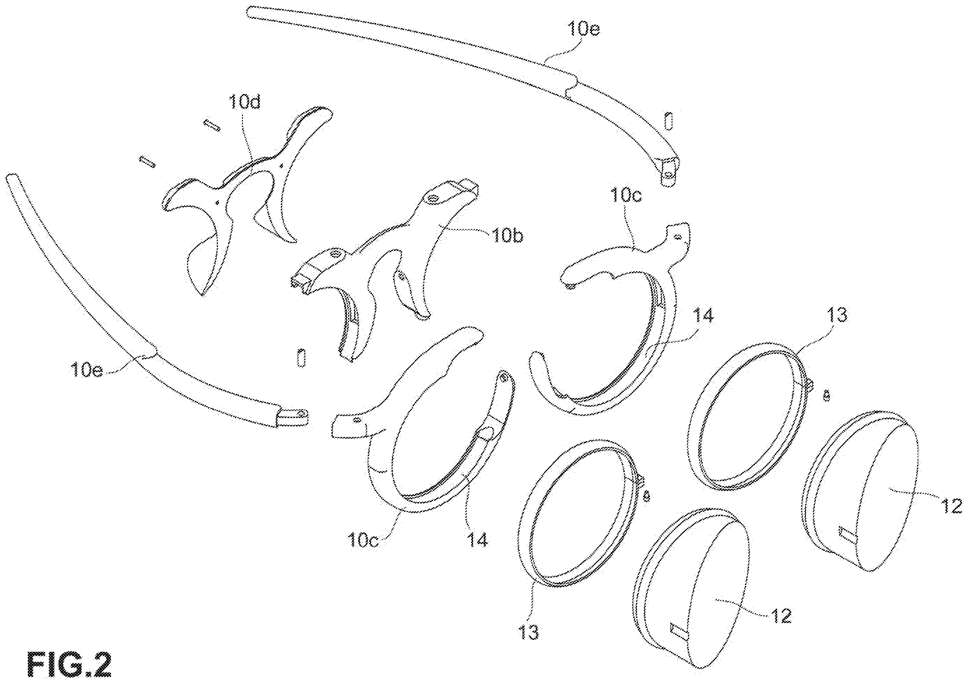

- figure 2 is an exploded schematic view of the optical instrument of figure 1;

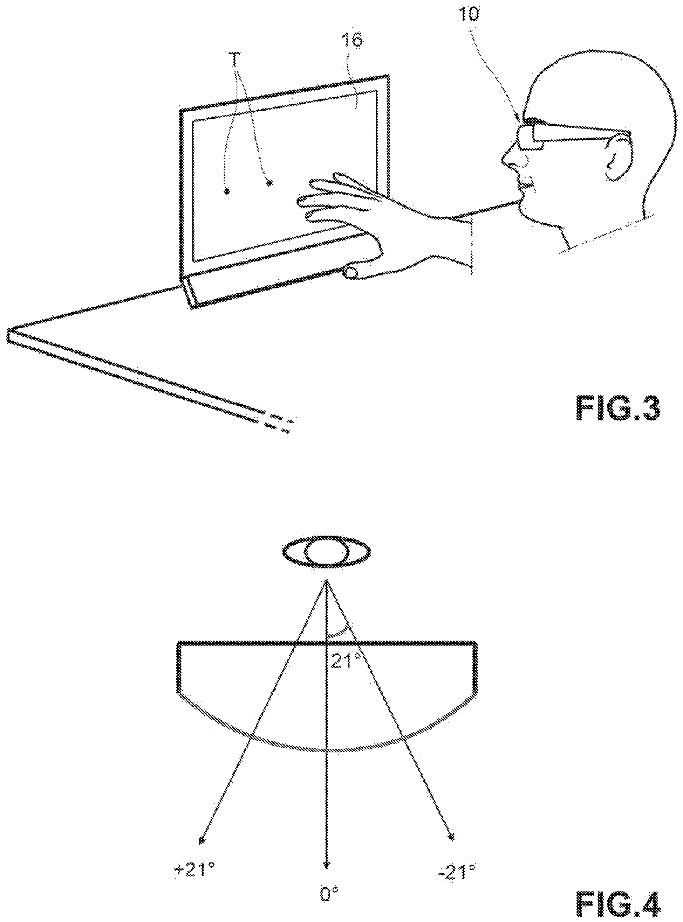

- figure 3 is a schematic view of a user who interfaces with a system according to an embodiment of the present invention;
- figure 4 is a schematic diagram of a possible way of generating the visual target stimuli, presented in this case along a visual arc of 42° with respect to the subject; and
- figures 5A and 5B are schematic diagrams of two possible ways of generating acoustic stimuli, respectively for a monaural and binaural stimulation of the subject.

### Detailed description

Before explaining a plurality of embodiments of the invention in detail, it should be noted that the invention is not limited in its application to the construction details and to the configuration of the components presented in the following description or shown in the drawings. The invention can take other embodiments and be implemented or practically carried out in different ways within the scope of protection set by the appended claims. It should also be understood that the phraseology and terminology are for descriptive purpose and are not to be construed as limiting.

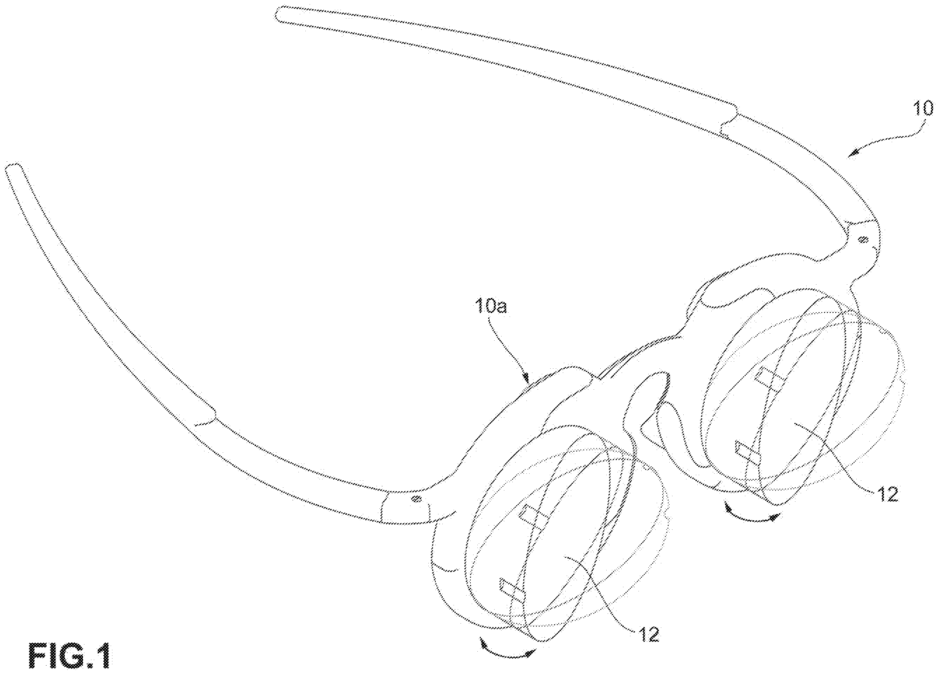

Referring by way of example to figure 1, a system 9 for enhancing or rehabilitating the cognitive skills of a subject comprises an optical instrument 10 wearable by such subject, including at least a prismatic lens 12 adapted to divert a beam of light rays coming from a visual target stimulus T in such a way as to induce a perturbation of the vision of said visual target stimulus T to the subject.

The wearable optical instrument 10 comprises a housing seat 14 for the at least one prismatic lens 12, adapted to rotatably support the latter.

Conveniently, the prismatic lens 12 may be coupled to a ring nut or ring 13, rotatably housed in the housing seat 14.

The system further comprises a screen 16 for displaying a visual target stimulus T, which may be a projection screen, a computer monitor or a tablet, etc.; an electronic image generation unit, programmed to generate a predetermined sequence of visual target stimuli T intended for focusing the subject's gaze, in a variable position on the area of the screen 16; aiming sensor means arranged to detect the aiming of the visual target stimulus by the subject (aiming which can be carried out by the subject for example by means of a mouse or finger); recording means, arranged for recording the aiming movements and/or aiming position by the subject in association with each visual target stimulus T; and processing means for determining the amount of deviation of said aiming position with respect to the displayed position of the visual target stimulus T. By way of example, such amount of deviation may be determined in degrees of visual angle, and/or may consist in detecting the side of the screen 16 towards which the subject's gaze deviates with respect to the displayed position of the visual target stimulus T (for example, assuming that the subject's gaze deviates more to the right or to the left with respect to the displayed position of the visual target stimulus T).

Storage means are also provided, adapted to store data indicative of a cognitive profile of the subject and of an orientation of the at least one prismatic lens 12 adapted to induce a vision perturbation of the visual target stimulus T to the subject associated with a predetermined therapeutic effect on the subject.

The processing means are arranged to determine which orientation of the at least one prismatic lens 12 is necessary to induce the predetermined therapeutic effect according to the cognitive profile of the subject. In other words, the processing means associate the cognitive profile with the orientation or adjustment of the prismatic lens 12 adapted to achieve a therapeutic effect functional to such a cognitive profile.

The subject's cognitive profile includes information indicative of his/her state of health and/or disorders felt by the subject, and may already be available before the procedure, so that the data is preloaded in the storage means, or the cognitive profile may be acquired by means of the procedure, so that the subject's response to the visual target stimulus T is processed by the processing means, which derive data indicative of the cognitive profile, which is then stored by the storage means. In general, the subject's cognitive profile is acquired through neuropsychological and cognitive tests, such as questions asked to the subject, examination of his/her clinical past, etc., and coded in a series of information that can be stored by the storage means.

A therapeutic effect to be achieved is therefore predetermined, according to the subject's cognitive profile (enhancement or rehabilitation of skills such as attention, language, motor skills, short and long-term memory, etc.); the desired therapeutic effect is associated with an orientation or degree of adjustment of the at least one prismatic lens 12, capable of inducing a corresponding perturbation of the vision of the visual target stimulus T to the subject (according to methods illustrated by way of example in the continuation of the present description). The processing means, by comparing the subject's cognitive profile with the desired therapeutic effect, determine the optimal orientation to be given to the at least one prismatic lens 12.

According to the invention, the processing means are arranged to compare the amount of deviation of the aiming position with respect to the displayed position of the visual target stimulus T, with the predetermined perturbation associated with a predetermined therapeutic effect on the subject as a function of the cognitive profile stored by the storage means. In this way, it becomes possible, for example, to acquire the subject's cognitive profile and to determine the orientation of the prismatic lenses 12 to achieve the desired therapeutic effect, and to verify the correspondence of the subject's reaction to the expected therapeutic effect and, if this correspondence is insufficient, the processing means could determine how the orientation of the at least one prismatic lens 12 should be modified.

According to the invention, the processing means are arranged to interact with the optical instrument 10 to orient the at least one prismatic lens 12 so as to induce said predetermined vision perturbation of the visual target stimulus T to the subject associated with a predetermined therapeutic effect. Actuator means (not shown) are therefore present, operated by the processing means and adapted to rotate the prismatic lens 12 in the respective housing seat 14. Conveniently, the electronic image generation unit is programmed to generate said visual target stimuli T alternatively in a central position on the area of the screen 16, in a position belonging to a first side portion of the screen 16 and in a position belonging to a second lateral portion of the screen 16 opposite to the first portion with respect to an axis of vertical symmetry.

The aiming sensor means may include a mouse or a tactile surface of the display screen 16. Moreover, the electronic image generation unit is controlled by said processing means to display a visual target stimulus T on the screen 16 or for a variable time or as long as said processing means determine a substantial coincidence of said aiming position with respect to the displayed position of the visual target stimulus T.

According to an embodiment, the at least one prismatic lens 12 is arranged to filter determined wavelengths of the beam of light rays coming from the visual target stimulus T. For example, the at least one prismatic lens 12 may be arranged to transmit a wavelength of said beam of light rays belonging to an interval of the electromagnetic spectrum corresponding to the red, orange or blue color. Preferably, the at least one prismatic lens 12 is arranged to filter only light radiation of wavelength in a range comprised between 620 and 750 nm or in a range comprised between 450 and 480 nm.

According to a preferred embodiment, the wearable optical instrument 10 is a pair of glasses, comprising a frame 10a which includes said housing seat 14 of said at least one prismatic lens 12. Conveniently, the frame 10a is configured to rotatably support a pair of prismatic lenses 12.

The frame 10a may comprise one or more of the following elements: a central body 10b, to which one or more lateral frames 10c which house the housing seats 14 are connected, an internal rubber 10d, which can be coupled to the central body 10b to increase the comfort of the wearer, and one or more temples 10e to support the lenses 12 once worn.

According to an alternative embodiment, the wearable optical instrument 10 is a virtual reality viewer with at least one integrated prismatic lens 12.

Conveniently, the visual stimuli T comprise at least one of circular images, letters of the alphabet, numbers, words, drawings.

According to an embodiment, the electronic image generation unit is arranged to transmit to the optical instrument 10 a signal corresponding to a visual target stimulus T with a frequency of 10 Hz or 40 Hz.

According to a further embodiment, the system comprises an electronic unit for generating an electromagnetic signal of an acoustic stimulus, arranged to transmit such electromagnetic signal of an acoustic stimulus to receiving means (not shown) coupled to the wearable optical instrument, which are adapted to convert the electromagnetic signal of an acoustic stimulus into at least one acoustic stimulus at at least one frequency audible by the subject, for conditioning the subject in a way complementary to the visual stimulus.

For example, the receiving means may be mountable on, or integrated in, one or both the temples 10e of the optical instrument 10, and may conveniently be powered by a battery which can in turn be incorporated in the temple 10e. The receiving means may be associated with a headset (for example, of the Bluetooth type) adapted to transmit the acoustic stimulus to the user's ear.

Optionally, the receiving means coupled to the wearable optical instrument are controlled to emit a monoaural acoustic stimulus and/or a binaural acoustic stimulus. According to one embodiment, the frequency audible by the subject is a frequency variable between 40 and 80 Hz.

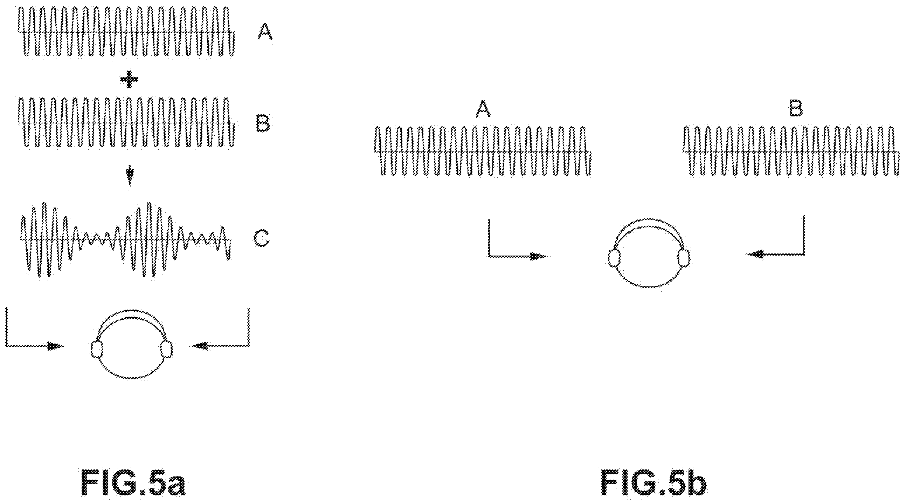

As shown by way of example in figures 5A and 5B, it is for example possible to obtain a left or right monaural stimulation (figure 5A) by transmitting to the receiving means sounds at different frequencies, for example a sound A at the frequency of 440 Hz, and a sound B at the frequency 480 Hz, so as to obtain a sound C at the resulting frequency of 40 Hz. Similarly, simultaneous binaural stimulation (figure 5B) may be obtained by transmitting sounds A, B at the frequency of 480 Hz. Again, a sound C will be obtained at the resulting frequency of 40 Hz.

Some examples of a rehabilitation procedure in which a system according to the present invention can be used, are given below, to illustrate more fully the effects and potential of this system.

For example, to enhance the excitability of the right cerebral hemisphere, it is possible to provide for the induction of a deviation of the visual field towards the right space, for example by rotating the prismatic lenses 12 so that the apices are located on the right side, that is, with the apex of the left prism in the nasal (internal) position and the apex of the right prism in the temporal (external) position. The adaptation step is carried out with aiming movements towards conveniently circular stimuli (for example, with a diameter of about 1° of visual angle). The post-adaptation step may always be carried out with aiming movements towards the same stimuli. In the post-adaptation step, the subject will have a deviation towards the left space, which causes an increase in the excitability of the right hemisphere.

Likewise, to enhance the excitability of the left cerebral hemisphere, it is possible to provide for the induction of a deviation of the visual field towards the left space, for example by rotating the lenses so that the apices are located on the left side, that is, with the apex of the left prism in the temporal (external) position and the apex of the right prism in the nasal (internal) position. The adaptation step is carried out with aiming movements towards circular stimuli (diameter: about 1° of visual angle). The post-adaptation step may always be carried out with aiming movements towards the same stimuli. In the post-adaptation step, the subject will have a deviation towards the right space, which causes an increase in the excitability of the left hemisphere.

It is also possible to induce an underestimation of time intervals, by inducing a deviation of the visual field as described above. The adaptation step may be carried out by presenting a conveniently circular stimulus (for example, with a diameter of about 1° of visual angle) in a central position with respect to the median sagittal plane of the subject's trunk, of fixed duration, randomly selected by software, and around 2000 ms. The subject is asked to make an aiming movement towards this stimulus. This stimulus is called "standard stimulus." The subsequent stimuli, with the same visual characteristics as the first, conveniently have a variable duration, randomly selected by software, lower or higher than the standard stimulus, with minimum differences of 200 ms and maximum of 400 ms, and are always presented centrally located. These stimuli are called "test" stimuli. In this procedure the subject will have to make aiming movements towards a fixed stimulus (for example a vertical bar, having a height of about 1° of visual angle) located in the left space (suitably, up to -21°) if he/she deems that the duration of the test stimulus is less than that of the standard stimulus, and towards a stimulus located in the right space (suitably, up to + 21°) if he/she deems that the duration of the test stimulus is greater than that of the standard stimulus.

The post-adaptation step may always be carried out with aiming movements towards the same stimuli, according to the procedure described above. In the post-adaptation step, the subject will have a deviation towards the left space, which will determine an effect of underestimation in time.

It is also possible to induce a facilitation of the phonological or semantic verbal fluidity by inducing a deviation of the visual field. The adaptation step is carried out with the induction of a deviation of the visual field towards the left space, for example by rotating the lenses so that the apices are located on the left side, that is, with the apex of the left prism in the temporal (external) position and the apex of the right prism in the nasal (internal) position. During and after the adaptation step, the subject will be able to perform verbal and phonological fluency tasks, composing words that begin with a specific letter of the alphabet or that correspond to a specific semantic category, by aiming movements towards various positions of the screen in which letters of the alphabet are displayed.

To induce a facilitation of verbal short-term memory, the adaptation step is carried out with the induction of a deviation of the visual field towards the left space, for example by rotating the lenses so that the apices are located on the left side, that is, with the apex of the left prism in the temporal (external) position and the apex of the right prism in the nasal (internal) position. During and after the adaptation step, the subject will be able to perform tasks of reproducing increasing sequences of numbers or symbols previously displayed, by aiming movements towards various positions of the screen in which the aforementioned stimuli are present.

To induce a spatial short-term memory facilitation, the adaptation step is carried out by the induction of a deviation of the visual field towards the right space, for example by rotating the lenses so that the apices are located on the right side, that is, with the apex of the left prism in the nasal (internal) position and the apex of the right prism in the temporal (external) position. During and after the adaptation step, the subject will be able to perform tasks of reproducing increasing sequences of spatial positions, identified by previously displayed visual stimuli, by aiming movements towards various screen positions corresponding to the coded spatial positions.

To induce a long-term verbal memory facilitation, or a neuro-immunomodulation process, the adaptation step is carried out by the induction of a deviation of the visual field towards the left space, for example by rotating the lenses so that the apices are located on the left side, that is, with the apex of the left prism in the temporal (external) position and the apex of the right prism in the nasal (internal) position. During and after the adaptation step, the subject will be able to perform reproduction/re-enactment or recognition of sequences of numbers, words, faces, buildings previously displayed in sequences that exceed the short-term memory span, by aiming movements to various positions of the screen in which the aforementioned stimuli to be held in memory are displayed.

For each of the proposed procedures, the software can automatically analyze the performance of the subjects in the proposed task, with or without glasses, and provide a score value both raw and corrected by age and schooling. For each test, the adjusted scores can therefore be displayed on a 5-level scale (from 0 to 4).

Various aspects and embodiments of a rehabilitation system according to the invention have been described. It is understood that each embodiment may be combined with any other embodiment. The invention, moreover, is not limited to the described embodiments, but may be varied within the scope defined by the appended claims.

## Claims

1. A system (9) for the cognitive enhancement or rehabilitation of a subject, comprising:
- a screen (16) for displaying a visual target stimulus (T);
- an electronic image generation unit, programmed to generate a predetermined sequence of visual target stimuli (T) intended for focusing the subject's gaze, in a variable position on the area of the screen (16), the visual target stimuli (T) covering about 1° of visual angle of the subject;
- aiming sensor means arranged to detect the aiming of the visual target stimulus (T) by the subject;
- an optical instrument (10) wearable by said subject, including at least one prismatic lens (12) adapted to divert a beam of light rays coming from the visual target stimulus (T) so as to induce a vision perturbation of the visual target stimulus (T) to the subject, said wearable optical instrument (10) further comprising a housing seat (14) for said at least one prismatic lens (12) adapted to rotatably support said prismatic lens (12);
- recording means, arranged for recording the aiming movements and/or aiming position by the subject in association with each visual target stimulus (T);
- processing means for determining the amount of deviation of said aiming position with respect to the displayed position of the visual target stimulus (T); and
- storage means, adapted to store data indicative of a cognitive profile of the subject and of an orientation of the at least one prismatic lens (12) adapted to induce a vision perturbation of the visual target stimulus (T) to the subject associated with a predetermined therapeutic effect on the subject;
said processing means being arranged to determine which orientation of the at least one prismatic lens (12) is necessary to induce the predetermined therapeutic effect according to the cognitive profile of the subject;
wherein the processing means are adapted to compare the amount of deviation of the aiming position with respect to the displayed position of the visual target stimulus (T), with the vision perturbation associated with a predetermined therapeutic effect on the subject as a function of the cognitive profile stored by the storage means;
**characterized in that** the processing means are arranged to interact with the optical instrument (10) to orient the at least one prismatic lens (12) so as to induce said vision perturbation of the visual target stimulus (T) to the subject associated with a predetermined therapeutic effect, the system (9) further comprising actuator means operated by the processing means and adapted to rotate the prismatic lens (12) in the respective housing seat (14), so as to modify the orientation of the prismatic lens (12) as long as the subject's reaction to the expected therapeutic effect is not sufficient.

2. A system according to claim 1, wherein the subject's cognitive profile includes information indicative of the subject's state of health and/or disturbances felt by the subject.

3. A system according to any one of the preceding claims, wherein said electronic image generation unit is programmed to generate said visual target stimuli (T) alternatively in a central position on the area of the screen (16), in a position belonging to a first side portion of the screen (16) and in a position belonging to a second lateral portion of the screen (16) opposite to the first portion with respect to an axis of vertical symmetry of the screen (16).

4. A system according to any one of the preceding claims, wherein said aiming sensor means include a mouse or a tactile surface of said display screen (16).

5. A system according to any one of the preceding claims, wherein said electronic image generation unit is controlled by said processing means to display a visual target stimulus (T) on the screen (16) for a variable time or as long as said processing means determine a substantial coincidence of said aiming position with respect to the displayed position of the visual target stimulus (T).

6. A system according to any one of the preceding claims, wherein said at least one prismatic lens (12) is arranged to filter determined wavelengths of said beam of light rays coming from the visual target stimulus (T).

7. A system according to claim 6, wherein said at least one prismatic lens (12) is arranged to filter only light radiation of wavelength in a range comprised between 620 and 750 nm or in a range comprised between 450 and 480 nm.

8. A system according to any one of the preceding claims, wherein said wearable optical instrument (10) is a pair of glasses, comprising a frame (10a) which includes said housing seat (14) of said at least one prismatic lens (12) adapted to rotatably support said prismatic lens (12).

9. A system according to any one of claims 1 to 7, wherein said wearable optical instrument (10) is a virtual reality viewer with at least one integrated prismatic lens (12).

10. A system according to any one of the preceding claims, wherein said visual target stimuli (T) comprise at least one of images of circular shape, letters of the alphabet, numbers, words, drawings.

11. A system according to any one of the preceding claims, wherein the electronic image generation unit is arranged to transmit to the optical instrument (10) a signal representative of a visual target stimulus (T) with a frequency of 10 Hz or 40 Hz.

## Patentansprüche

1. System (9) zur kognitiven Förderung oder Rehabilitation eines Subjekts, umfassend:
- einen Bildschirm (16) zum Anzeigen eines visuellen Zielreiz (T);
- eine elektronische Bilderzeugungseinheit, die programmiert wird, um eine vorbestimmte Folge von visuellen Zielreizen (T), die dazu bestimmt sind, den Blick des Subjekts in einer variablen Position in dem Bereich des Bildschirms (16) zu fokussieren, wobei die visuellen Zielreize (T) etwa 1° des Sehwinkels des Subjekts abdecken;
- Orientierungssensormittel, die eingerichtet sind, die Orientierung des visuellen Zielreizes (T) durch das Subjekt zu erkennen;
- ein optisches Instrument (10), das von dem Subjekt tragbar ist, enthaltend mindestes eine prismatische Linse (12), die angepasst ist, ein Lichtstrahlbündel, das von dem visuellen Zielreiz (T) ausgeht, abzulenken, um eine Sehstörung des visuellen Zielreizes (T) in dem Subjekt zu veranlassen, wobei das tragbare optische Instrument (10) ferner einen Aufnahmesitz (14) für die mindestens eine prismatische Linse (12) umfasst, der eingerichtet ist, die prismatische Linse (12) drehbar zu halten;
- Aufzeichnungsmittel, die eingerichtet sind, die Orientierungsbewegungen und/oder die Orientierungsposition des Subjekts in Verbindung mit jedem visuellen Zielreiz (T) aufzuzeichnen;
- Verarbeitungsmittel zum Bestimmen des Wertes der Ablenkung der Orientierungsposition in Bezug auf die angezeigte Position des visuellen Zielreizes (T); und
- Speichermittel, die eingerichtet sind, Daten zu speichern, die ein kognitives Profil des Subjekts und eine Orientierung der mindestens einer prismatischen Linse (12) anzeigen, die eingerichtet ist, eine Sehstörung des visuellen Zielreizes in dem Subjekt (T) zu veranlassen, die einer vorbestimmten therapeutischen Wirkung auf das Subjekt zugeordnet ist;
wobei die Verarbeitungsmittel eingerichtet sind, zu bestimmen, welche Orientierung der mindestens einer prismatischen Linse (12) erforderlich ist, um die vorbestimmte therapeutische Wirkung nach dem kognitiven Profil des Subjekts zu veranlassen;
wobei die Verarbeitungseinheiten eingerichtet sind, den Wert der Ablenkung der Orientierungsposition in Bezug auf die angezeigte Position des visuellen Zielreizes (T) mit der Sehstörung zu vergleichen, die einer vorbestimmten therapeutischen Wirkung auf das Subjekt in Abhängigkeit von dem durch die Speichermittel gespeicherten kognitiven Profil zugeordnet ist;
**dadurch gekennzeichnet, dass** die Verarbeitungsmittel eingerichtet sind, mit dem optischen Instrument (10) zu interagieren, um die mindestens eine prismatische Linse (12) zu orientieren, sodass die Sehstörung des visuellen Zielreizes (T) in dem Subjekt zu veranlassen, die einer vorbestimmten therapeutischen Wirkung zugeordnet ist, das System (9) ferner umfassend Aktuatormittel, die von den Verarbeitungsmitteln betrieben werden und die eingerichtet sind, die prismatische Linse (12) in dem jeweiligen Aufnahmesitz (14) zu drehen, um die Orientierung der prismatischen Linse (12) zu ändern, solange die Reaktion des Subjekts auf die erwartete therapeutische Wirkung nicht ausreichend ist.

2. System nach Anspruch 1, wobei das kognitive Profil des Subjekts Informationen umfasst, die den Gesundheitszustand des Subjekts und/oder die von dem Subjekt empfundene Störungen anzeigen

3. System nach einem der vorhergehenden Ansprüche, wobei die elektronische Bilderzeugungseinheit programmiert wird, visuellen Zielreize (T) abwechselnd in einer Zentralposition auf dem Bereich des Bildschirms (16), in einer Position, die zu einem ersten seitlichen Abschnitt des Bildschirms (16) gehört, und in einer Position, die zu einem zweiten seitlichen Abschnitt des Bildschirms (16) gehört, gegenüber dem ersten Abschnitt in Bezug auf eine vertikale Symmetrieachse des Bildschirms (16), zu erzeugen.

4. System nach einem der vorhergehenden Ansprüche, wobei die Orientierungssensormittel eine Maus oder eine Berührungsfläche des Anzeigebildschirms (16) umfassen.

5. System nach einem der vorhergehenden Ansprüche, wobei die elektronische Bilderzeugungseinheit von den Verarbeitungsmitteln gesteuert wird, um einen visuellen Zielreiz (T) auf dem Bildschirm (16) für eine variable Zeit oder so lange anzuzeigen, bis die Verarbeitungsmittel eine wesentliche Übereinstimmung der Orientierungsposition in Bezug auf die angezeigte Position des visuellen Zielreizes (T) bestimmen.

6. System nach einem der vorhergehenden Ansprüche, wobei die mindestens eine prismatische Linse (12) dazu eingerichtet ist, bestimmte Wellenlängen des Lichtstrahlbündel, das von dem visuellen Zielreiz (T) ausgeht, zu filtern.

7. System nach Anspruch 6, wobei die mindestens eine prismatische Linse (12) dazu eingerichtet ist, nur Lichtstrahlung mit einer Wellenlänge im Bereich zwischen 620 und 750 nm oder in einem Bereich zwischen 450 und 480 nm zu filtern.

8. System nach einem der vorhergehenden Ansprüche, wobei das tragbare optische Instrument (10) eine Brille ist, umfassend einen Rahmen (10a), der den Aufnahmesitz (14) der mindestens einer prismatischen Linse (12) enthält, der eingerichtet ist, die prismatische Linse (12) drehbar zu halten.

9. System nach einem der Ansprüche 1 bis 7, wobei das tragbare optische Instrument (10) ein virtueller Reality-Betrachter mit mindestens einer integrierten prismatischen Linse (12) ist.

10. System nach einem der vorhergehenden Ansprüche, wobei die visuellen Zielreize (T) mindestens eines der folgenden umfassen: Bilder mit kreisförmiger Form, Buchstaben des Alphabets, Zahlen, Wörter, Zeichnungen.

11. System nach einem der vorhergehenden Ansprüche, wobei die elektronische Bilderzeugungseinheit dazu eingerichtet ist, ein Signal, das einen visuellen Zielreiz (T) darstellt, mit einer Frequenz von 10 Hz oder 40 Hz an das optische Instrument (10) zu übertragen.

## Revendications

1. Système (9) pour l'amélioration ou la rééducation cognitive d'un sujet, comprenant :
- un écran (16) pour l'affichage d'un stimulus visuel cible (T) ;
- une unité électronique de génération d'images, programmée pour générer une séquence prédéterminée de stimuli visuels cibles (T) destinés à focaliser le regard du sujet, dans une position variable sur la zone de l'écran (16), les stimuli visuels cibles (T) couvrant environ 1° d'angle visuel du sujet ;
- des moyens capteurs de visée disposés pour détecter la visée du stimulus visuel cible (T) par le sujet ;
- un instrument optique (10) portable par ledit sujet, comprenant au moins une lentille prismatique (12) apte à dévier un faisceau de rayons lumineux provenant du stimulus visuel cible (T) de manière à induire une perturbation de la vision du stimulus visuel cible (T) chez le sujet, ledit instrument optique portable (10) comprenant en outre un logement (14) pour ladite au moins une lentille prismatique (12) apte à supporter ladite lentille prismatique (12) de manière rotative ;
- des moyens d'enregistrement, disposés pour enregistrer les mouvements de visée et/ou la position de visée par le sujet en association avec chaque stimulus visuel cible (T) ;
- des moyens de traitement pour déterminer la quantité de déviation de ladite position de visée par rapport à la position affichée du stimulus visuel cible (T) ; et
- des moyens de stockage, aptes à stocker des données indicatives d'un profil cognitif du sujet et d'une orientation de ladite au moins une lentille prismatique (12) apte à induire une perturbation de la vision du stimulus visuel cible (T) chez le sujet associé à un effet thérapeutique prédéterminé sur le sujet ;
lesdits moyens de traitement étant disposés pour déterminer quelle orientation de ladite au moins une lentille prismatique (12) est nécessaire pour induire l'effet thérapeutique prédéterminé selon le profil cognitif du sujet ;
les moyens de traitement étant aptes à comparer la quantité de déviation de la position de visée par rapport à la position affichée du stimulus visuel cible (T), avec la perturbation de la vision associée à un effet thérapeutique prédéterminé sur le sujet en fonction du profil cognitif stocké par les moyens de stockage ;
**caractérisé en ce que** les moyens de traitement sont disposés pour interagir avec l'instrument optique (10) pour orienter ladite au moins une lentille prismatique (12) de manière à induire ladite perturbation de la vision du stimulus visuel cible (T) chez le sujet associée à un effet thérapeutique prédéterminé, le système (9) comprenant en outre des moyens d'actionnement commandés par les moyens de traitement et aptes à faire tourner la lentille prismatique (12) dans le logement respectif (14), de manière à modifier l'orientation de la lentille prismatique (12) tant que la réaction du sujet à l'effet thérapeutique attendu n'est pas suffisante.

2. Système selon la revendication 1, dans lequel le profil cognitif du sujet comprend des informations indicatives de l'état de santé du sujet et/ou des troubles ressentis par le sujet.

3. Système selon l'une quelconque des revendications précédentes, dans lequel ladite unité électronique de génération d'images est programmée pour générer lesdits stimuli visuels cibles (T) alternativement en position centrale sur la zone de l'écran (16), en une position appartenant à une première portion latérale de l'écran (16) et en une position appartenant à une seconde portion latérale de l'écran (16) opposée à la première portion par rapport à un axe de symétrie verticale de l'écran (16).

4. Système selon l'une quelconque des revendications précédentes, dans lequel lesdits moyens capteurs de visée comprennent une souris ou une surface tactile dudit écran d'affichage (16).

5. Système selon l'une quelconque des revendications précédentes, dans lequel ladite unité électronique de génération d'images est commandée par lesdits moyens de traitement pour afficher un stimulus visuel cible (T) sur l'écran (16) pendant un temps variable ou aussi longtemps que lesdits moyens de traitement déterminent une coïncidence substantielle de ladite position de visée par rapport à la position affichée du stimulus visuel cible (T).

6. Système selon l'une quelconque des revendications précédentes, dans lequel ladite au moins une lentille prismatique (12) est disposée pour filtrer certaines longueurs d'onde dudit faisceau de rayons lumineux provenant du stimulus visuel cible (T).

7. Système selon la revendication 6, dans lequel ladite au moins une lentille prismatique (12) est disposée pour filtrer uniquement les radiations lumineuses de longueur d'onde comprise entre 620 et 750 nm ou comprise entre 450 et 480 nm.

8. Système selon l'une quelconque des revendications précédentes, dans lequel ledit instrument optique portable (10) est une paire de lunettes, comprenant une monture (10a) qui inclut ledit logement (14) de ladite au moins une lentille prismatique (12) apte à supporter ladite lentille prismatique (12) de manière rotative.

9. Système selon l'une quelconque des revendications 1 à 7, dans lequel ledit instrument optique portable (10) est un dispositif de visualisation en réalité virtuelle avec au moins une lentille prismatique (12) intégrée.

10. Système selon l'une quelconque des revendications précédentes, dans lequel lesdits stimuli visuels cibles (T) comprennent au moins l'un parmi des images de forme circulaire, des lettres de l'alphabet, des chiffres, des mots, des dessins.

11. Système selon l'une quelconque des revendications précédentes, dans lequel l'unité électronique de génération d'images est disposée pour transmettre à l'instrument optique (10) un signal représentatif d'un stimulus visuel cible (T) avec une fréquence de 10 Hz ou 40 Hz.
